# EUROPEAN PATENT APPLICATION

(11) **EP 1 832 282 A1**
(43) Date of publication of application: **12.09.2007**
(21) Application number: 05814720.8
(22) Date of filing: 08.12.2005
(51) Int. Cl.: A61K 9/48, A61K 47/32, A61K 47/36, A61J 3/07, C08L 31/04, C08L 39/06

(54) **BAND SEAL FOR HARD CAPSULE**

(30) Priority: 28.12.2004 JP 2004379309
(71) Applicant: Qualicaps Co., Ltd., Nara 639-1032 (JP)
(72) Inventor: TOCHIO, Shinji, c/o QUALICAPS CO., LTD., Yamatokoriyama-shi, Nara 6391032 (JP); NAGATA, Shunji, 7370112 (JP); SAKUMA, Satoshi, Ohtsu-shi, Shiga 520-0802 (JP)
(74) Representative: Findeisen, Marco
(86) International application number: PCT/JP2005/022547
(87) International publication number: WO 2006/070578

(57) **Abstract**

The present invention has as its object to provide a band-seal to manufacture a leak-tight, stable hard capsule preparation not being susceptible to deformation after having filled pharmaceuticals, foods, healthy foods, etc. in a hard capsule comprising a polyvinyl alcohol copolymer or pullulan as a base, and in more particular, relates to a band-seal intended for use in the hard capsule comprising a polyvinyl alcohol copolymer or pullulan as a base, **characterized in that** the band-seal is incorporated with at least one or not less than two of the below-described (a) to (c):
(a) A polyvinylpyrrolidone with a molecular weight of 100,000 to 4,000,000,
(b) A polyvinylpyrrolidone with a molecular weight of 10,000 to 80,000 in the proportion of not more than 90% by weight against the total weight of the band-seal; and
(c) A copolymer from 1-vinyl-2-pyrrolidone and vinyl acetate.

## Description

### Technical Field

The present invention relates to a band-seal intended for use in the hard capsule comprising a polyvinyl alcohol copolymer or pullulan as a base which is incorporated with at least one or not less than two of (a) a polyvinylpyrrolidone with a molecular weight of 100,000 to 4 , 000 , 000 , (b) a polyvinylpyrrolidone with a molecular weight of 10,000 to 80,000 in the proportion of not more than 90% by weight against the total weight of the band-seal and (c) a copolymer from 1-vinyl-2-pyrrolidone and vinyl acetate.

### Background Art

Band-sealing for hard capsules is ordinarily effected with gelatin for hard capsules made of gelatin or with hydroxypropylcellulose (HPMC) for hard capsules of HPMC, simply because use of the same material in band-sealing as the material of construction for hard capsules can allow mutual dissolution of the material at the portion of sealing, thus yielding the potent bonding.

Gelatin is sparingly soluble in water at room temperature, and, when dissolved in water through heating at increased temperatures (ca. 50°C) and used for sealing for hard capsules of gelatin, can be prevented from becoming soluble in water used for said sealing by instantaneously cooling the sealing portion.

On the other hand, pullulan and polyvinyl alcohol copolymers are soluble in water and, when aqueous solutions of such materials are used in the sealing, leave the resultant hard capsule partly vulnerable to dissolution even after cooling the sealing portion. In order to avoid such phenomena, there has been adopted the procedure which involves admixing water with ethyl alcohol to accelerate the moisture drying rate.

However, difficulties are encountered in dissolving pullulan in a solvent mixture consisting of water and ethyl alcohol, whereas polyvinyl alcohol copolymers are soluble in a solvent mixture of water and ethyl alcohol but requires a prolonged length of time to dry the sealed portion, causing the resultant hard capsules themselves to be deformed.

There have been disclosed so far in the past a hard capsule of pullulan and a process for manufacturing the same (Official Gazettes of Published Japanese Translation of the PCT Publication No. 2003-505565 A and of JP No.2000-202003 A) and a capsule of a polyvinyl alcohol copolymer and a process for manufacturing the same (The Pamphlet of WO 02/17848), but neither of these prior art literature references has described anything about the method of conducting sealing for such hard capsules of pullulan or polyvinyl alcohol copolymer.

Furthermore, the Official Gazettes of JP Nos. Sho 59-105455 A and Sho 57-1344 A disclosed methods of sealing hard capsules which comprises allowing a sealing liquid to be absorbed by capillarity into a gap between a body and cap of the hard capsule. But these processes are faced with the problem that a sealing liquid, when applied inadequately to a gap between the cap and body parts to thereby allow for absorption, makes the appearance extremely dirty.

Additionally, the Official Gazette of JP No. Sho 59-115047 A described a method of sealing a capsule and a device therefor, but delineates that a gluing adhesive agent is adhered to the inner surface of a capsule being kept spaced from the edge of a cap and introducing a specified amount of the product into a body of the said capsule, followed by engagement of the said body containing the product introduced into the cap having the said adhesive belt applied, thereby making it likely to stain the appearance, as is the case with the prior art literature references of JP Nos. Sho 59-105455 A and Sho 57-1344 A.

### Disclosure of the Invention

### [The Problem That the Invention Is Intended to Solve]

There has been existing a demand for the supply of a leak-tight sealed hard capsule in various industrial sectors, such as the ones of pharmaceuticals, foods, healthy foods, cosmetics, agrochemicals, etc. A solid drug substance, when it is sensible to oxygen or water, is vulnerable to possible degradation with the oxygen or water penetrating within the capsule through a gap between its cap and body, and consequently, there is also demanded a hard capsule almost tightly sealed against penetration of oxygen or water through a gap between its cap and body. With reference to the countermeasure to solve such problems, various proposals have been made so far in the past, but can be said to be still far from satisfactory. The object of the present invention is to provide a band-seal for hard capsules which is effective for the prevention of liquid leakage and also to provide a hard capsule being obtainable with use of said band-seal which capsule is free from liquid leakage, nearly impenetrable to oxygen and water, and resistant to deformation in the sealed portion, especially a capsule comprising a base consisting of a polyvinyl alcohol copolymer or pullulan which capsule can reduce the risk of involvement of BSE (bovine spongiform encephalopathy).

As used herein, the term "seal" is understood to comprehend "sealing" as the case maybe, and the term "band-seal" is intended to denote a solid-formed portion to be created by sealing the cap and body of a capsule with a solution for preparing the band-seal, followed by drying, while the expression "solution for preparing the band-seal" is understood to signify a liquid component in which a ingredient composed mainly of the band-seal before formation of the band-seal and additives are brought into dissolution or suspension.

### [Means for Solving the Problem]

The present inventors conducted intensive investigation to solve the problem, and as a result, discovered a band-seal intended for use in the hard capsule comprising a polyvinyl alcohol copolymer or pullulan as a base, which is incorporated with at least one or not less than two of (a) a polyvinylpyrrolidone with a molecular weight of 100,000 to 4,000,000, (b) a polyvinylpyrrolidone with a molecular weight of 10,000 to 80,000 in the proportion of not more than 90% by weight against the total weight of the band-seal or (c) a copolymer from 1-vinyl-2-pyrrolidone and vinyl acetate, leading to completion of the present invention.

Thus, the present invention relates to:
(1) A band-seal intended for use in the hard capsule comprising a polyvinyl alcohol copolymer or pullulan as a base,
   characterized in that the band-seal is incorporated with at least one or not less than two of the below-described (a) to (c):
   (a) A polyvinylpyrrolidone with a molecular weight of 100,000 to 4,000,000;
   (b) A polyvinylpyrrolidone with a molecular weight of 10,000 to 80,000 in the proportion of not more than 90% by weight against the total weight of the band-seal; and
   (c) A copolymer from 1-vinyl-2-pyrrolidone and vinyl acetate,
(2) The band-seal as described above under (1), wherein the polyvinyl alcohol copolymer is contained in the band seal at a proportion of not more than 60% by weight against the total weight of the band seal,
(3) A solution for preparing the band-seal intended for use in the hard capsule comprising a polyvinyl alcohol copolymer or pullulan as a base, wherein the solution for preparing a band-seal comprises at least one or not less than two of the below-described (a) to (c):
   (a) A polyvinylpyrrolidone with a molecular weight of 100,000 to 4,000,000;
   (b) A polyvinylpyrrolidone with a molecular weight of 10,000 to 80,000; and
   (c) A copolymer from 1-vinyl-2-pyrrolidone and vinyl acetate, and (a) and (c) are contained in the proportion of 3 to 35% by weight against the total weight of the solution for preparing the band-seal and (b) is contained in the proportion of 8 to 40% by weight against the total weight of the solution for preparing the band-seal,
(4) The solution for preparing the band-seal as described above under (3) , wherein the polyvinyl alcohol copolymer is contained in the proportion of not more than 15% by weight against the total weight of the solution for preparing the band-seal,
(5) The solution for preparing the band-seal as described above under (3) or (4), wherein the said solution for preparing the band-seal shows a viscosity of 100 to 5,500 mPa · s,
(6) The band-seal or the solution for preparing the band-seal as described above under any one of (1) to (3), characterized in that the copolymer from 1-vinyl-2-pyrrolidone and vinyl acetate is composed of 1-vinyl-2-pyrrolidone and vinyl acetate at the weight ratio of 4:1 to 1:4,
(7) The band-seal or the solution for preparing the band-seal as described above under (2) or (4), characterized in that the polyvinyl alcohol copolymer is produced by copolymerizing a partially saponified polyvinyl alcohol with a mean degree of polymerization of 300 to 500 and a polymerizable vinyl monomer at the weight ratio of 6:4 to 9 : 1, with the polymerizable vinyl monomer being composed of acrylic acid and methyl methacrylate at the weight ratio of 3:7 to 0.5:9.5,
(8) The band-seal or the solution for preparing the band-seal as described above under (2) or (4), characterized in that the polyvinyl alcohol copolymer is obtained by copolymerizing a partially saponified polyvinyl alcohol with a mean degree of polymerization of 300 to 500 with methyl methacrylate and acrylic acid at the weight ratio of 60 to 90:7 to 38:0.5 to 12,
(9) A hard capsule which is sealed with the band-seal as described under any one of (1), (2), and (6) to (8),
(10) A method for sealing a hard capsule, characterized in that said method comprises sealing a hard capsule with the band-seal as described under any one of (1), (2), and (6) to (8),
(11) A hard capsule which has a liquid-formed or solid-formed substance filled therein, followed by sealing with the band-seal as described under any one of (1), (2), and (6) to (8),
(12) A hard capsule which has a liquid-formed or solid-formed drug substance filled therein, followed by sealing with the band-seal as described under any one of (1), (2), and (6) to (8),
(13) A hard capsule, characterized in that said hard capsule is produced by filling a liquid-formed or solid-formed substance therein and sealing the hard capsule with the solution for preparing the band-seal as described under any one of (3) to (8), followed by drying, and
(14) A process for producing a hard capsule preparation,
characterized in that said process comprises filling a liquid-formed or solid-formed substance in a hard capsule, and sealing the hard capsule with the solution for preparing the band-seal as described under any one of (3) to (8), followed by drying.

### [Effect of the Invention]

The band-seal of the present invention can be effectively used for producing the hard capsules which can ensure hermetical sealing of the cap and body portions so that the liquid material as filled in may not leak out of the hard capsule, while at the same time, the solid-formed or liquid-formed substance as filled in the hard capsule may be prevented from degradation through its contact with air or moisture. Also, the band-seal of the present invention, which contributes not only to diminish wastes owing to defects of the product but also to facilitate the process control, can reduce the production time and production cost and is suited for the large-scale, mass production. The band-seal of the present invention being intended for use in the hard capsule comprising a polyvinyl alcohol copolymer or pullulan as a base, characterized in that said band-seal is incorporated with at least one or not less than two of the below-described (a) to (c):
(a) A polyvinylpyrrolidone with a molecular weight of 100,000 to 4,000,000,
(b) A polyvinylpyrrolidone with a molecular weight of 10,000 to 80,000 in the proportion of not more than 90% by weight against the total weight of the band-seal, and
(c) A copolymer from 1-vinyl-2-pyrrolidone and vinyl acetate, can effectively prevent liquid leakage from the hard capsule after having a liquid content filled therein, and also can suppress degradation of the filled substance which is vulnerable to degradation with oxygen or moisture, while it does not cause the hard capsule to be deformed at its sealed portion. Furthermore, the band-seal of the present invention remains free from embrittlement and stays tough even after forming the sealing (band sealing).

The solution for preparing a band-seal to form the band-seal being intended for use in a hard capsule comprising a polyvinyl alcohol copolymer or pullulan as a base comprises at least one or not less than two of the below-described (a) to (c):
(a) A polyvinyl pyrrolidone with a molecular weight of 100,000 to 4,000,000,
(b) A polyvinylpyrrolidone with a molecular weight of 10,000 to 80,000, and
(c) A copolymer from 1-vinyl-2-pyrrolidone and vinyl acetate, wherein (a) and (c) are contained in the proportion of 3 to 35% by weight against the total weight of the solution for preparing the band-seal and (b) is contained in the proportion of 8 to 40% by weight against the total weight of the solution for preparing the band-seal, and, when the polyvinyl alcohol copolymer is incorporated in the proportion of not more than 15% by weight against the total weight of the solution for preparing the band-seal and the said solution for preparing the band-seal shows a viscosity of 100 to 5500 mPa · s, can act to prevent the hard capsule effectively from liquid leak after having had a substance filled therein and also the filled substance from degradation, and additionally can produce the band-seal which does not cause the hard capsule to be deformed at the sealed portion. The said solution for preparing a band-seal, which displays the lessened tendency (a degree of thread drawing) to draw threads, is easy to handle in the manufacturing process. On the other hand, the hard capsule preparation as produced with use of the band-seal according to the present invention shows improved processability, and can be made fit for ordinary transference and smooth transportation without causing liquid leakage, while needing not to take any special countermeasure for transference and transportation.

### [Brief Description of the Drawing]

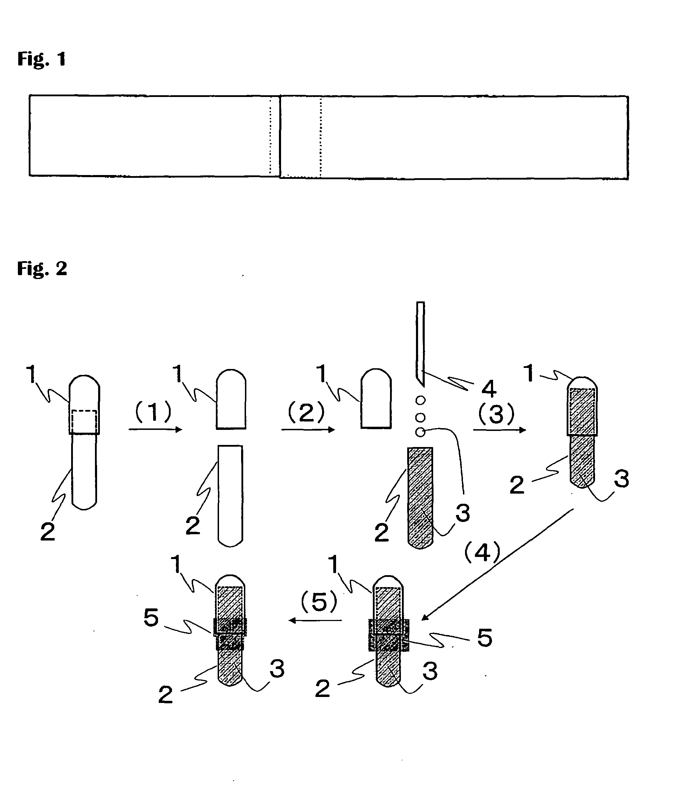

Fig. 1 is a method of adhesion in the adhesion test for a cast film according to PONDAC, wherein adhesion is effected within the region encircled by a dotted line;
Fig. 2 is a schematic drawing of a process for manufacturing the hard capsule preparation of the present invention, wherein the step (1) is a step of detaching or separating the body and cap parts of a hard capsule; the step (2) is a step of filling a substance (the contents) to be filled into the body part of a hard capsule; the step (3) is a step of engaging the cap part into the body part of a hard capsule containing the filled contents; the step (4) is a step of sealing the engaged hard capsule with the solution of preparing a band-seal; and the step (5) is a step of drying the sealed hard capsule.

### [Remarks of the Signs]

1: Cap part of a hard capsule
2: Body part of a hard capsule
3: Contents
4: Filling tube
5: Sealed portion (band-seal portion)

### [The Best Mode for Carrying out the Invention]

The band-seal of the present invention is characterized in that said band-seal, which is incorporated with at least one or not les s than two of (a) a polyvinylpyrrolidone with a molecular weight of 100,000 to 4,000,000, (b) a polyvinylpyrrolidone with a molecular weight of 10,000 to 80,000 in the proportion of not more than 90% by weight against the total weight of the band-seal, and (c) a copolymer from 1-vinyl-2-pyrrolidone and vinyl acetate, is a band-seal intended for use in the hard capsule comprising a polyvinyl alcohol copolymer or pullulan as a base.

In the context of this, one kind of the polyvinylpyrrolidones to be used as a high-molecular component of the band-seal shows a molecular weight in the range of ca. 100,000 to 4,000,000, preferably ca. 500,000 to 2,000,000, furthermore preferably ca. 900,000 to 1,500,000. Any polyvinylpyrrolidones, only if they show such molecular weight ranges, do not involve the danger of getting embrittled after completion of the band-seal, and may possibly be utilizable practically as a band-seal. Their specific examples include Polyvinylpyrrolidone K-90 (with a weight-average molecular weight of ca. 1,200,000; produced by Wako Pure-Chemical Co. of Japan), etc. and, in order to enhance the band-seal strength and also to reduce the thread drawing tendency of the solution for preparing a band- seal, furthermore, there is preferably mingled a polyvinylpyrrolidone having a smaller weight-average molecular weight than the above-mentioned, whereby the weight-average molecular weight of polyvinylpyrrolidones is to be determined in accordance with the descriptions given in Handbook of Pharmaceutical excipients (4th edition, Edited by R. C. Rowe et al., Pharmaceutical Press, 2003).

The amount of a polyvinylpyrrolidone (e.g., a polyvinylpyrrolidone with a weight-average molecular weight of ca. 1,200,000) having such molecular-weight distribution of 100,000 to 4,000,000 to be formulated is in proportions of 40 to 100% by weight against the total weight of the band-seal, preferably 45 to 100% by weight, more preferably 50 to 100% by weight. Such polyvinylpyrrolidones, when formulated in smaller proportions than the above-described ones, bring about the danger of failing to form the strong or tough band-seal.

The miscellaneous polyvinylpyrrolidones preferably include polyvinylpyrrolidones having a molecular-weight distribution of 10,000 to 80,000, specifically Polyvinylpyrrolidone K-15 (with a weight-average molecular weight of: ca. 10,000; produced by Wako Pure-Chemical Co. of Japan), Polyvinyl- pyrrolidone K-25 (with a weight-average molecular weight of: ca. 25,000; produced by Wako Pure-Chemical Co. of Japan) and Polyvinylpyrrolidone K-30 (with a weight-average molecular weight of: ca. 40 , 000 ; produced by Wako Pure-Chemical Co. of Japan), preferably Polyvinylpyrrolidone K-25.

The amount of such polyvinylpyrrolidones (e.g., polyvinylpyrrolidone with a weight-average molecular weight of ca. 25,000) with a molecular weight distribution of 10,000 to 80,000 to be formulated is in proportions of not more than 90% by weight against the total weight of the band-seal, preferably 40 to 90% by weight , more preferably 45 to 90% by weight, furthermore preferably 50 to 90% by weight. Such polyvinylpyrrolidones, when formulated in smaller proportions than the above-defined ones, involves the danger of causing the thread drawing, resulting in difficulties in processing the band-seal, and, when formulated in greater proportions, allows the resulting solution for preparing a band-seal to reduce its viscosity so excessively as to prevent the solution from being applied on the capsule in adequate amounts and also as to provide the resultant band-seal with embrittlement, leading to inability to be utilized practically as a band-seal. In the above context, the expression "the total weight of the band-seal" is understood to denote the total weight on a solid weight basis of the dried band-seal.

The copolymer (b) from 1-vinyl-2-pyrrolidone and vinyl acetate includes, for example, copolyvidone (tradename: Plasdone S-630, produced by ISP Japan Co., a copolymer from 1-vinyl-2-pyrrolidone and vinyl acetate at amonomerweight ratio of 60:40), as specified in Standard for Additives for Pharmaceutical Preparations (2003), etc. The above-mentioned copolyvidone shows a weight-average molecular weight of generally 5,000 to 1, 000 , 000 , preferably 7 , 500 to 500 , 000 , more preferably 10,000 to 100,000.

The amount of the copolymers from 1-vinyl-2-pyrrolidone and vinyl acetate to be formulated is in proportions of 40 to 100% by weight against the total weight of the band-seal, preferably 45 to 100% by weight, more preferably 50 to 100% by weight. Such copolymers, when formulated in smaller proportions than the above-defined ones, yield merely the brittle band-seal and may be impossible to be utilized practically as a band-seal.

In the copolymers from 1-vinyl-2-pyrrolidone and vinyl acetate, the weight ratio of 1-vinyl-2-pyrrolidone and vinyl acetate ranges from 4:1 to 1:4, preferably 3:2. The copolymer, when the weight of 1-vinyl-2-pyrrolidone exceeds the ratio of 4: 1, may exhibit an increased viscosity, whereas the weight of vinyl acetate in excess of 1:4 results in a possibility that the resultant band-seal, becoming readily soluble in water, fails to function as a band-seal.

If desired, a polyvinyl alcohol copolymer can furthermore be incorporated into the band-seal. The polyvinyl alcohol copolymer includes, for example, polyvinyl alcohol copolymers as obtained by copolymerizing a polyvinyl alcohol or its derivative with a polymerizable vinyl monomer, preferably high-molecular copolymers produced by copolymerizing a partially saponified polyvinyl alcohol as a skeleton with acrylic acid and methyl methacrylate, and the like, more preferably polyvinyl alcohol copolymers obtained by copolymerizing partially saponified polyvinyl alcohol with a mean polymerization degree of 300 to 500 with a polymerizable vinyl monomer at a weight ratio of ca. 6:4 to 9:1, wherein the said polymerizable vinyl monomer is acrylic acid and methyl methacrylate and their weight ratio on the occasion of copolymerization is in the range of ca. 3:7 to 0.5:9.5. Particularly preferable are polyvinyl alcohol copolymers obtained by copolymerizing a partially saponified polyvinyl alcohol with a mean degree of polymerization 300 to 500 with methyl methacrylate and acrylic acid at the weight ratio of 60 to 90:7 to 38:0.5 to 12. Specifically, such copolymers can be exemplified by PONDAC (the registered trademark: PONDAC; produced by Nisshin Chemical Co. of Japan). This copolymer can be produced in accordance with the procedure as described in the Pamphlet of WO 02/17848.

The polyvinyl alcohol copolymers cannot be used solely as a band-seal, but are usable as a mixture with some high molecular substance. Referring to the high molecular substance to be mixed with the polyvinyl alcohol copolymers, the above-described polyvinylpyrrolidones or copolymers from 1 -vinyl- 2 -pyrrolidone and vinyl acetate can be mixed. The mixing ratio based on the total weight of the band-seal is 0.1 to 60% by weight of the polyvinyl alcohol copolymer to 40 to 99.9% by weight of the polyvinylpyrrolidone, or 0.1 to 60% by weight of the polyvinyl alcohol copolymer to 40 to 99.9% by weight of the copolymer from 1-vinyl-2-pyrrolidone and vinyl acetate.

The band-seal, which is usable in the production of a band-seal for the hard capsule comprising a polyvinyl alcohol copolymer or pullulan as a base, can constitute firm and strong band-seals, only if it is incorporated with at least either (a) a polyvinylpyrrolidone containing a polyvinylpyrrolidone (e.g., Polyvinylpyrrolidone K-90, or a polyvinylpyrrolidone with a weight-average molecular weight of ca. 1,200,000) with a molecular weight of 100,000 to 4,000,000 in proportions of 10 to 50% by weight against the total weight of polyvinylpyrrolidone or (b) a copolymer from 1-vinyl-2 - pyrrolidone and vinyl acetate, and if desired, may be furthermore incorporated with a polyvinyl alcohol copolymer, provided, however, that the solution for preparing a band-seal is incorporated with the polyvinylpyrrolidone with a molecular weight of 100,000 to 4,000,000 in proportions of less than 15% by weight against the total weight of the solution for preparing a band-seal and shows a viscosity of 100 to 5,500mPa· s. It is to be added that the said solution is free from thread-drawing and easy to be handled during the production process.

The solution for preparing a band-seal shows a viscosity of 100 to 5,500mPa·s, preferably 125 to 5,400mPa · s, more preferably 150 to 5,300mPa · s. The solution for preparing a band-seal, when it shows a viscosity lower than the above-specified range, cannot be applied on a hard capsule, there in some instances results the fear that no band-seal could be formed. The solution, when it shows a viscosity greater than the above-specified range, runs the risk that no band-seal could be formed by the machine owing to increased viscosity.

The solution for preparing a band-seal, even when it shows a viscosity in the above-specified range, if it is incorporated with a polyvinylpyrrolidone (e.g., Polyvinylpyrrolidone K-90, or a polyvinylpyrrolidone with a weight-average molecular weight of ca. 1,200,000) with a molecular weight of 100 , 000 to 4,000,000 in proportions of greater than 35% by weight against the total weight, develops the thread-drawing tendency, resulting in the fear that the band-seal forming treatment would be difficult. On the other hand, the solution for preparing a band-seal, if it is incorporated with a polyvinylpyrrolidone with a molecular weight of 100,000 to 4,000,000 in proportions of less than 3% by weight against the total weight, is liable to provide brittle band-seals. Consequently, the solution for preparing a band-seal is to be incorporated with a polyvinylpyrrolidone with a molecular weight of 100,000 to 4,000,000 in proportions of 3 to 35% by weight against the total weight of the solution for preparing a band-seal, preferably 4 to 32.5% by weight, more preferably 5 to 30% by weight.

The solution for preparing a band-seal is incorporated with a polyvinylpyrrolidone (e.g., Polyvinylpyrrolidone K-25, or a polyvinylpyrrolidone with a molecular weight of ca. 25,000) with a molecular weight of 10,000 to 80,000 in proportions of 8 to 40% by weight against the total weight, preferably 8.5 to 37.5% by weight, furthermore preferably 9 to 35% by weight, as described in the above. The solution for preparing a band-seal, when the proportion of the polyvinylpyrrolidone is smaller than the above-specified range, is liable to provide brittle band-seals, whereas the solution, with its content of the polyvinylpyrrolidone in excess of the above-specified range, may show an increased viscosity and be difficult to be handled during the production process.

As described in the above, the solution for preparing a band-seal, when it is incorporated through mixing with a polyvinylpyrrolidone (hereinafter referred to sometimes as "PVP-A", being exemplified by Polyvinylpyrrolidone K-90, or a polyvinylpyrrolidone with a weight-average molecular weight of ca. 1,200,000) with a molecular weight of 100,000 to 4,000,000 and a polyvinylpyrrolidone (hereinafter referred to sometimes as "PVP-B" , being exemplified by Polyvinylpyrrolidone K-25, or a polyvinylpyrrolidone with a weight-average molecular weight of ca. 25,000) with a molecular weight of 10,000 to 80,000, can also produce the firm an strong band-seals which are easy to handle during production process. The optimal formulation amount or ratio each of PVP-A and PVP-B in the solution for preparing a band-seal are: 1) 5% by weight of PVP-A and 25 to 40% by weight of PVP-B, 2) 7.5% by weight of PVP-A and 25 to 30% by weight of PVP-B, 3) 10% by weight of PVP-A and 20 to 30% by weight of PVP-B, and 4) 12.5% by weight of PVP-A and 15 to 25% by weight of PVP-B, and PVP-A and PVP-B can be mixed at the above-mentioned ratios to produce a solution for preparing a band-seal, followed by sealing of a capsule by the specifically determined procedure and drying to thereby form a band-seal.

With reference to the formulation amount of the copolymer from 1-vinyl-2-pyrrolidone and vinyl acetate in the solution for preparing a band-seal, such copolymer is formulated in proportions of 3 to 35% by weight against the total weight of the solution, preferably 4 to 32.5% by weight, furthermore preferably 5 to 30% by weight. When the formulation amount is less than the above-defined range, the resultant solution for preparing a band-seal shows a decreased viscosity, being unable to be applied on a hard capsule and also to form a band-seal, and needs to be applied in larger quantities, thus requiring a prolonged length of time for drying and being liable to deform the capsule. When the formulation amount is greater than the above-defined range, the resultant solution for preparing a band-seal shows a too much increased viscosity, thus being liable to fail to form a band-seal with a machine.

Referring to the formulation amount of the polyvinyl alcohol copolymer in the solution for preparing a band-seal, such copolymer is formulated in proportions of 0 to 22.5% by weight against the total weight of the solution, preferably 0 to 20% by weight, furthermore preferably 0 to 17.5% by weight. When the formulation amount is greater than the above-defined range, the resultant solution for preparing a band-seal shows a too much increased viscosity, thus being liable to fail to form a band-seal with a machine.

The above-described high molecular compounds are dissolved in a solvent to make the solution for preparing a band-seal, whereby the above-described high molecular compounds can be suitably formulated in an appropriate solvent consisting of a sole solvent or a combination of not less than two solvents at room temperature or under warming (at ca. 30 to 60°C) to give a solution for preparing a band-seal. The solvent for the solution for preparing a band-seal may be exemplified by water, alcohols (e.g., ethanol) or their mixtures, and is preferably mixtures of water and alcohols which permit the drying step to be simplified. The amount of such solvent mixture as used is at ratios of ca. 10 to 90% by weight of water to ca. 10 to 90% by weight of an alcohol against the whole of the solution for preparing a band-seal, while the mixing ratio of water to alcohol is generally ca. 1:4 to 4 : 1, particularly preferably ca. 2:3.

It is possible to color the band-seal with a coloring agent, such as titanium oxide, iron oxide red, coal-tar based coloring matters, etc. Such coloring agents may not be particularly limited, only if they have been approved for use in pharmaceuticals, foods or cosmetics, etc.

The band-seal according to the present invention can be applied for effecting the sealing of the body and cap parts of a hard capsule having a polyvinyl alcohol copolymer or pulullan utilized as a base.

The hard capsule having a polyvinyl alcohol copolymer utilized as excipient can be prepared in accordance with the description given in WO 02/17848. Namely, the pins each for forming a capsule are soaked in the solution for preparing a capsule consisting of a polyvinyl alcohol copolymer and a gelling agent dissolved in an appropriate solvent (e.g., water, an alcohol, etc.), and then drawn up to thereby allow the capsule-preparing solution as adhered to the pins to undergo gelling and drying on the pins.

In this context, the polyvinyl alcohol copolymer is as produced by copolymerizing a polyvinyl alcohol and a vinyl monomer, such as methacrylic acid, methyl methacrylate, acrylic acid, etc., and may be used as an additive for pharmaceuticals, cosmetics, etc., wherein use can be made of any commercially available ones, such as Pondac (produced by Nisshin Kasei Co. of Japan).

On the occasion of this, the concentration of the polyvinyl-alcohol copolymer in the capsule-preparing solution ranges from 5 to 30% by weight, preferably from 6 to 25% by weight, furthermore preferably from 7 to 20% by weight.

The gelling agent, which is added to the solution for preparing a hard capsule comprising a polyvinyl alcohol copolymer, is preferably any substances being susceptible to gelling by changes in temperature, and their specific examples include xanthan gum, locust bean gum, gelan gum, carrageenan, tamarind seed polysaccharide, pectin, curdlan, gelatin, furcelleran, agar, etc. , wherein use can furthermore be made of such substances in the form of mixtures of not less than two kinds thereof.

Among others, xanthan gum, locust bean gum, gelan gum, and carrageenan are suited for the polyvinyl alcohol copolymer, and particularly, use of gelan gum can yield rapidly soluble capsules. The concentration of the above-described gelling agent in the capsule preparing solution is ca. 0.01 to 10% by weight, preferably ca. 0.05 to 5% by weight, more preferably ca. 0.1 to 1.0% by weight.

In cases where κ-carrageenan is used as a gelling agent for hard capsules based on a polyvinyl alcohol copolymer, meanwhile, as a gelling adjuvant, use can be made of water-soluble compounds capable of yielding potassium, ammonium and calcium ions, such as potassium chloride, ammonium chloride, ammonium acetate, calcium chloride, etc. In case where L-carrageenan is used as a gelling agent, meanwhile, as a gelling adjuvant, use can be made of water-soluble compounds capable of yielding calcium ion, such as calcium chloride, etc. In cases where gelan gum is utilized as a gelling agent, also, as a gelling adjuvant, use can be made of water-soluble compounds capable of yielding sodium, potassium, calcium and magnesium ions, such as sodium chloride, potassium chloride, calcium chloride, magnesium sulfate, etc. and furthermore, there can also be utilized organic acids and their water-soluble salts, such as citric acid or sodium citrate.

When use is made of the above-described gelling adjuvant, additionally, it is preferable to use such gelling adjuvant at ratios of ca. 0.05 to 0.6% by weight in the capsule-preparing solution, preferably ca. 0.055 to 0.4% by weight, more preferably ca. 0.06 to 0.2% by weight.

It is to be added that on the occasion of manufacturing hard capsules based on a polyvinyl alcohol copolymer, there may be added suitable amounts of additives ordinarily used for hard capsules, such as coloring agents, opacifying agents, fragrances, etc.

The soaking temperature for the pins for hard capsules based on a polyvinyl alcohol copolymer may be desirably selected and is preferably set at ca. 30 to 80° C, preferably ca. 35 to 70° C, more preferably ca. 40 to 60°C.

The solution for preparing a capsule preferably shows ca. 100 to 5,000Pa · s (as measured by the B-type revolving viscometer) in viscosity during pin-soaking. When the viscosity is lower than the above-specified range, there is brought about a decrease in the amount of the starting material for capsules as adhered to a pin for forming a capsule, thereby being liable to provide the resultant capsule with a reduced film thickness, whereas a too much increased viscosity in excess of the range leads to the possibility that the shape of the resulting capsule would generally be difficult to be controlled.

After drawing up the pins, gelling is preferably effected by leaving them on cooling, but may be conducted by heating for drying at ca. 40 to 80°C after gelling occurs.

The hard capsule comprising pullulan as a base can be manufactured in accordance with the description given in JP 2000-202003 A. Namely, the pin for forming a capsule is soaked in a solution for preparing capsules and then drawn up to thereby allow the solution for preparing a capsule as adhered onto the pin to gel and dry.

As used herein, the term "pullulan" refers to a naturally occurring polysaccharide having a repeating unit of maltotriose as joined through α-1,6-glucoside linkage as produced by culturing *Aureobasidium pullulans ,* a kind of black yeasts, with use of starch as a starting material, which has heretofore been known as an additive for foods, pharmaceuticals, cosmetics, etc. and use can be made of any commercially available ones, such as Pullulan PI-20 (produced by K.K. Hayashibara).

In such a case, the concentration of pullulan in the above-described solution for forming a capsule ranges from 5 to 30% by weight, preferably from 7.5 to 27.5% by weight, more preferably from 10 to 25% by weight.

The gelling agent to be added to the solution for forming hard capsule based on pullulan is preferably any substances which undergo readily gelling with changes in temperature, and their specific examples include xanthan gum, locust bean gum, gelan gum, carrageenan, tamarind seed polysaccharide, pectin, curdlan, gelatin, furcelleran, agar, etc. , which are usable as a mixture of not less than two kinds thereof.

Among others, xanthan gum/locust bean gum, gelan gum, and carrageenan are suited for pullulan, and particularly, use of gelan gum can yield rapidly soluble capsules . The concentration of the above-described gelling agent in the capsule preparing solution is ca. 0.01 to 10% by weight, preferably ca. 0.05 to 5% by weight, more preferably ca. 0.1 to 1.0% by weight.

In cases where κ-carrageenan is used as a gelling agent for hard capsules based on pullulan, meanwhile, as a gelling adjuvant, use can be made of water-soluble compounds capable of producing potassium, ammonium and calcium ions, such as potassium chloride, ammonium chloride, ammonium acetate, calcium chloride, etc. In case where 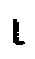 -carrageenan is used as a gelling agent, meanwhile, as a gelling adjuvant, use can be made of water-soluble compounds capable of yielding calcium ion, such as calcium chloride, etc. In cases where gelan gum is utilized as a gelling agent, also, as a gelling adjuvant, use can be made of water-soluble compounds capable of yielding sodium, potassium, calcium and magnesium ions, such as sodium chloride, potassium chloride, calcium chloride, magnesium sulfate, etc. and furthermore, there can also be utilized organic acids and their water-soluble salts, such as citric acid or sodium citrate.

When use is made of the above-described gelling adjuvant, additionally, it is preferable to use such gelling adjuvant at ratios of ca. 0.05 to 0.6% by weight in the capsule-preparing solution, preferably ca. 0.055 to 0.4% by weight, more preferably ca. 0.06 to 0.2% by weight.

It is to be added that on the occasion of manufacturing hard capsules based on pullulan, there may be added suitable amounts of additives ordinarily used for hard capsules, such as coloring agents, opacifying agents, fragrances, etc.

The soaking temperature for the pins for hard capsules based on pullulan may be desirably selected and is preferably set at ca. 30 to 80° C, preferably ca. 35 to 70° C, more preferably ca. 40 to 60°C.

The solution for preparing a capsule preferably shows ca. 100 to 5,000Pa·s (as measured by the B-type revolving viscometer) in viscosity during soaking. When the viscosity is lower than the above-specified range, there is brought about a decrease in the amount of the starting material for capsules as adhered to a pin for forming a capsule, thereby being liable to provide the resultant capsule with a reduced film thickness, whereas a too much increased viscosity in excess of the range leads to the possibility that the shape of the resulting capsule would generally be difficult to be controlled.

After drawing up the pins, gelling is preferably effected by leaving them on cooling, but, may be conducted by heating for drying at ca. 40 to 80°C after gelling occurs.

Fig. 1 illustrates schematically a process for producing a hard capsule and a hard-capsule preparation according to the present invention. The body part (2) and cap part (1) of the hard capsule can be produced individually from a larger-sized pin for forming capsule and a smaller-sized one. The thus-produced body and cap parts of a hard capsule based on a polyvinyl alcohol copolymer or pullulan are joined through engagement after filling the contents (3) in the body part through a filling tube (4), and the engaged portion can be subjected to sealing (5) by applying once to several times, preferably once to twice, a band-seal onto the surface each of the body and cap parts at their respective circular zones of a width having the end edge of the cap part as a center and in their radial directions. Prior to sealing of the engaged portion, the contents (e.g., pharmaceuticals, foods, healthy foods) can be filled in the body part in accordance with the procedure known *per* se to produce a hard capsule preparation.

The contents or substances to be filled, which are to be filled in the hard capsule of the present invention, include for example medicinal drugs, foods, healthy foods for human or animal, etc., and any substances can be subjected to filling, unless they dissolve the hard-capsule films made of a polyvinyl alcohol copolymer or pullulan or react with such hard-capsule film. The contents may be in any forms and states, inclusive of solids, semi-solids, crystals, oils, prepared liquids and the like. The hard capsule of the present invention can find suited application not only as a container for oral administration of drug pharmaceuticals, foods, healthy foods, etc., but also as a capsule for enclosing the so-called quasi drug products being intended for use in disinfecting or washing false teeth, glasses, contact lenses, etc.

As contents to be filled in the hard capsule, the substances which are vulnerable to attack by oxygen or water can also be filled in such hard capsule, whereby there is created the possibility that the contents will be stabilized, because the band- seal as provided between the cap and body parts of the capsule can prevent oxygen or water from permeating through a cap between the cap and body parts.

The liquid fillings include, for example, alcohols and polyhydric alcohols, such as stearyl alcohol, cetanol, and polyethylene glycols 200, 400, 600, 800, 1000, 1500, 2000, 3000, 4000, 6000, 8000 and 20000, etc., oils and fats, such as sesame oil, soybean oil, peanut oil, corn oil, hardened oil, paraffin oil, bleached beeswax, etc., and fatty acids and their derivatives, such as stearic acid, palmitic acid, myristic acid, triethyl citrate, triacetin, middle-chain fatty acid triglycerides, and the like. It is to be noticed that the above-described liquid contents may be incorporated through dissolution with active substances, such as drugs, or may be incorporated through suspension with active substances and such liquid contents as such may in some instances constitute a solution of active substances.

As the pharmaceuticals to be filled in the capsule of the present invention, for example, use is made of one or not less than two kinds of drug(s) selected from nourishment tonics, antipyretics/analgesics/anti-inflammatory drugs, psychotropic drugs, anti-anxiety drugs, antidepressant drugs, hypnotic/sedative drugs, antispasmodic drugs, drugs acting on the central nervous system, cerebral metabolism improvers, cerebral circulation improvers, antiepileptic drugs, sympathetic nerve stimulants, digestives, antacids, antiulcer drugs, antitussive/expectorant drugs, antiemetic drugs, respiration promoters, bronchodilators, antiallergic drugs, drugs for dentistry and oral cavity, antihistamic drugs, cardiotonic drugs, antiarrhythmic drugs, diuretic drugs, antihypertensive drugs, vasoconstrictors, coronary vasodilators, peripheral vasodilators, antihyperlipidemic drugs, cholagogues, antibiotics, chemotherapeutic drugs, antidiabetic drugs, antiosteoporotic drugs, antirheumatic drugs, skeletal muscle relaxants, spasmolytic drugs, hormone preparations, alkaloid narcotics, sulfadrugs, anti-gout drugs, anticoagulant drugs, antineoplastic drugs and the like.

Examples of the nourishment tonics include vitamins, such as vitamins A, D, Es (e.g., d-α-tocopherol acetate, etc.), B₁s (e.g., dibenzoylthiamine,fursultiamine hydrochloride, etc.), B₂s (e.g., riboflavin butyrate, etc.), B₆s (e.g., pyridoxine hydrochloride, etc.), Cs(e.g., ascorbic acid, sodium L-ascorbate) and B₁₂S (e.g., hydroxocobalamin acetate, cyanocobalamin, etc.), minerals, such as calcium, magnesium, iron, etc., proteins, amino acids, oligosaccharides, herbal medicines and the like. The antipyretic/analgesic/anti-inflammatory drugs include, for example, aspirin, acetaminophen, ethenzamide, ibuprofen, diphenhydramine hydrochloride, dl-chlorpheniramine maleate, dihydrocodeine phosphate, noscapine, methylephedrine hydrochloride, phenylpropanolamine hydrochloride, caffeine, anhydrous caffeine, serrapeptase, lysozyme chloride, tolfenamic acid, mefenamic acid, diclofenac sodium, flufenamic acid, salicylamide, aminopyrine, ketoprofen, indomethacin, bucolome, pentazocine and the like.

The psychotropic drugs include, for example, chlorpromazine, reserpine and the like. The antianxiety or anxiolytic drugs may be exemplified by alprazolam, chlordiazepoxide, diazepam and the like. Examples of the antidepressant drugs include imipramine, maprotiline hydrochloride, amphetamine and the like. The hypnotic/sedative drugs may be exemplified by estazolam, nitrazepam, diazepam, perlapine, phenobarbital sodium and the like. The antispasmodic drugs include, for example, scopolamine hydrobromide, diphenhydramine hydrochloride, papaverine hydrochloride and the like. The drugs acting on the central nervous system may be exemplified by citicoline and the like. The cerebral metabolism improvers include, for example, meclofenoxate hydrochloride and the like. The cerebral circulation improvers include, for example, vinpocetine and the like. The antiepileptic drugs include, for example, phenytoin, carbamazepine and the like. The sympathetic nerve stimulants may be exemplified by isoproterenol hydrochloride and the like. The digestives include, for example, stomach digestives, such as diastase, saccharated pepsin, scopolia extract, cellulase AP3, lipase AP and cinnamon oil, intestinal regulators, such as berberine hydrochloride, resistant lactic bacteria and *Lactobacillus bifidus* and the like.

The antacids include, for example, magnesium carbonate, sodium hydrogencarbonate, magnesium aluminate metasilicate, synthetic hydrotalcite, precipitated calcium carbonate, magnesium oxide and the like. The antiulcer drugs may be exemplified by lansoprazole, omeprazole, rabeprazole, famotidine, cimetidine, ranitidine hydrochloride and the like. The antitussive/expectorant drugs include, for example, cloperastine hydrochloride, dextromethorphan hydrobromides, theophylline, potassium guaiacolsulfonate, guaifenesin, codeine phosphate and the like. The antiemetic drugs include, for example, diphenidol hydrochloride, metoclopramide and the like. The respiration promoters include, for example, levallorphan tartrate and the like. The bronchodilators include, for example, theophylline, salbutamol sulfate and the like. The antiallergic drugs include, for example, amlexanox, seratrodast and the like. The drugs for dentistry and oral cavity may be exemplified by oxytetracycline, triamcinolone acetonide, chlorhexidine hydrochloride, lidocaine and the like.

The antihistaminic drugs include, for example, diphenhydramine hydrochloride, promethazine, isothipendyl hydrochloride, dl-chlorpheniramine maleate and the like. The cardiotonics include, for example, caffeine, digoxin and the like. The antiarrhythmic drugs include, for example, procainamide hydrochloride, propranolol hydrochloride, pindolol and the like. The diuretic drugs include, for example, isosorbide, furosemide, hydrochlorothiazide and the like. The antihypertensive drugs include, for example, delapril hydrochloride, captopril, hydralazine hydrochloride, labetalol hydrochloride, manidipine hydrochloride, candesartan cilexetil, methyldopa, perindopril erbumine and the like. The vasoconstrictors include, for example, phenylephrine hydrochloride and the like.

The coronary vasodilators include, for example, carbocromen hydrochloride, molsidomine, verapamil hydrochloride and the like. The peripheral vasodilators include, for example, cinnarizine and the like. The antihyperlipidemic drugs include, for example, cerivastatin sodium, simvastatin, pravastatin sodium, atorvastatin calcium hydrate and the like. The cholagogues include, for example, dehydrocholic acid, trepibutone and the like. The antibiotics include, for example, cephem antibiotics, such as cephalexin, cefaclor, amoxicillin, pivmecillinam hydrochloride, cefotiam hexetil hydrochloride, cefadroxil, cefixime, cefditoren pivoxil, cefteram pivoxil and cefpodoxime proxetil, synthetic antibacterial drugs, such as ampicillin, ciclacillin, nalidixic acid and enoxacin, monobactam antibiotics, such as carumonam sodium, and penem and carbapenem antibiotics, and the like.

The chemotherapeutic drugs include, for example, sulfamethizole and the like. The antidiabetic drugs include, for example, tolbutamide, voglibose, pioglitazone hydrochloride, glibenclamide, troglitazone and the like. The antiosteoporotic drugs include, for example, ipriflavone and the like. The skeletal muscle relaxants include, for example, methocarbamol and the like. The antispasmodic drugs include, for example, meclizine hydrochloride, dimenhydrinate and the like. The antirheumatic drugs include, for example, methotrexate, bucillamine and the like. The hormone preparations include, for example, liothyronine sodium, dexamethasone sodium phosphate, prednisolone, oxendolone, leuprorelin acetate and the like. The alkaloid narcotics include, for example, opium, morphine hydrochloride, ipecacuanha, oxycodon hydrochloride, opium alkaloid hydrochloride, cocaine hydrochloride and the like. The sulfa drugs include, for example, sulfisomidine, sulfamethizole and the like. The anti-gout drugs include, for example, allopurinol, colchicine and the like. The anticoagulants include, for example, dicumarol and the like. The antineoplastic drugs include, for example, 5-fluorouracil, uracil, mitomycin and the like.

These medicinal drugs can be used singly or as a combination preparation with other medicinal drugs, and are appropriately filled in the known suitable amount in the capsules, depending upon the disease, age, etc. of the patient.

The hard capsules are available in different sizes, e.g. Size Nos. 00, 0, 1, 2, 3, 4, 5 and the like, and in the present invention, the hard capsule having any Size Nos. can be produced and used, wherein preferable are the capsules as set forth in Japanese Pharmacopeia, European Pharmacopeia and U.S. Pharmacopeia.

The filling of the contents in the hard capsules can be performed by use of the per se known capsule-filling machines, such as a full-automatic capsule-filling machine (Model name: LIQFIL super 80/ 150 , fabricated by Shionogi Qualicaps Co. of Japan), a capsule-filing/sealing machine (Model name: LIQFIL super FS, fabricated by Shionogi Qualicaps Co. of Japan), a capsule-sealing machine (Model name: HICAPSEAL 40/100, fabricated by Shionogi Qualicaps Co. of Japan), etc.

In making engagement of the body and cap parts of the hard capsule, in general, the width of the engaging portion where the outer periphery of the body part overlaps with the inner periphery of the cap part preferably is in the range of ca. 4.5 to 6.5 mm for Size No. 3 capsule and ca. 4.0 to 6.0 mm for Size No. 4 capsule in the axis line direction of the capsule, whereby the sealing width generally is preferably ca. 1.5 to 5.0 mm as measured from the end of the cap part taken as the center.

The band-seal of the present invention can generally be used at room temperature or under warming, and the band-seal being kept at temperatures in the range of preferably ca. 10 to 60°C, more preferably ca. 20 to 40° C, can produce favorable effects on the prevention of liquid leaking from the hard capsule or prevention of the sealed portion from deforming. The band-seal can be subjected to temperature controlling by the per se known method using a panel heater, warm-water heater, etc. , and it is preferable to control the temperature with use of a circulating-type warm-water heater or a circulating-type warm-water heater as modified from a seal-pan unit of the above-mentioned one-piece (built-in) type capsule sealing machine, because such heater can permit the subtle regulation of temperature width. Since an alcohol in the band-seal, such as ethanol, vaporizes depending upon the ambient temperature, additionally, it is desirable to appropriately supplement such alcohol to maintain the composition of the band-seal to the constant one.

The above-described band-seal enables hard capsules to be sealed completely, thus providing an effective means for sealing hard capsules.

### Examples

The present invention is to be specifically illustrated by way Examples, Control Examples and Experiment Examples, but the present invention is not understood to be limited thereto.

### Example 1:

A ca. 270 mg quantity of polyethylene glycol 400 was filled into a hard capsule of Size No. 2 comprising a polyvinyl alcohol copolymer (a polyvinyl alcohol/acrylic acid/methyl methacrylate copolymer, produced by Nisshin Chemical Co. and Daidoh Chemical Co. of Japan) as a base. There was prepared a sealing solution (a solution for preparing the band-seal) composed of 10% by weight of a polyvinyl alcohol copolymer, 15% by weight of a copolymer from 1-vinyl-2-pyrrolidone and vinyl acetate (Copolyvidone, tradename of Plasdone S-630, produced by ISP Japan Co.), 40% by weight of ethyl alcohol and 35% by weight of water, and LAB Sealer (manufactured by Schaefer Technologies, Inc.) was employed to seal the hard capsule at room temperature, followed by drying at 40°C for ca. 15 min.

### Example 2:

A ca. 270 mg quantity of polyethylene glycol 400 was filled into a hard capsule of Size No. 2 comprising a polyvinyl alcohol copolymer (a polyvinyl alcohol/acrylic acid/methyl methacrylate copolymer, produced by Nisshin Chemical Co. and Daidoh Chemical Co. of Japan) as a base. There was prepared a sealing solution (a solution for preparing the band-seal) composed of 30% by weight of a copolymer from 1-vinyl-2-pyrrolidone and vinyl acetate (Copolyvidone, tradename of Plasdone S-630, produced by ISP Japan Co.), 50% by weight of ethyl alcohol and 20% by weight of water, and LAB Sealer (manufactured by Schaefer Technologies, Inc.) was employed to seal the hard capsule at room temperature, followed by drying at 40°C for ca. 15 min.

### Example 3:

A ca. 600 mg quantity of Gelucire (the trademark: produced by Gattesfosses Co.) was filled into a hard capsule of Size No. 0 comprising a pullulan (tradename: PI-20, produced by K.K. Hayashibara of Japan) as a base, and 35% by weight of a polyvinylpyrrolidone (comprising 5 % by weight and 30% by weight each of Povidone K9 0 and K25 as set forth in Japanese Pharmacopeia, 14^{th} revised edition) was dissolved in a solution consisting of 44% by weight of ethyl alcohol and 21% by weight of water. The solution was used and to seal the pullulan-based hard capsule having Gelucire filled therein with HICAPSEAL40 (manufactured by Shionogi Qualicaps Co. of Japan).

### Control Example 1

A ca. 600 mg quantity of Gelucire was filled into a hard capsule of Size No. 0 comprising a pullulan (tradename: PI-20, produced by K.K. Hayashibara of Japan) as a base. 35% by weight of pullulan was dissolved in 65% by weight of water, and the solution was used to seal the pullulan-based hard-capsule having Gelucire filled therein with HICAPSEAL40 (manufactured by Shionogi Qualicaps Co. of Japan).

### Assessment:

The hard capsules of Examples 1, 2 and 3 each showed a clean appearance at their sealed portions, with no leak being observed from the filled substance.

In Control Example 1, a solvent mixture system of ethyl alcohol and water was not able to be used, since pullulan was found to be sparingly soluble in ethyl alcohol, and consequently, pullulan was dissolved in water to carry out a sealing processing work, whereby drying was retarded, resulting in markedly deformation of the capsule.

### Example 4 :

A ca. 250 mg quantity of Polysorbate 80 was filled in a hard capsule of Size No. 3 comprising a polyvinyl alcohol copolymer (a polyvinyl alcohol/acrylic acid/methyl methacrylate copolymer, produced by Nisshin Chemical Co. and Daidoh Chemical Co. of Japan) as a base with use of LIQFIL-Super 40 (manufactured by Shionogi-Qualicaps Co. of Japan). There were prepared two kinds of solution for preparing the band-seals (sealing solutions) being composed of a polyvinyl alcohol copolymer and copolyvidone as shown in below Table 1, and HICAPSEAL-40 (manufactured by Shionogi-Qualicaps Co. of Japan) was employed to seal the hard capsule at room temperature (at the sealing rate of 20000 capsules/hr).

**Table 1:**

| Compositions of the sealing solutions for the hard capsule based on a polyvinyl alcohol copolymer | | |
|---|---|---|
| | Formulation 4-1 | Formulation 4-2 |
| Polyvinyl alcohol copolymer | 10% by wt. | - |
| Copolyvidone | 20% by wt. | 30% by wt. |
| Ethyl alcohol | 30% by wt. | 40% by wt. |
| Water | 40% by wt. | 30% by wt. |

### Assessment:

In Example 4, testing was conducted with the filling and sealing machines as employed in the commercial production, where 800 and 400 capsules were sealed with the sealing solutions of formulation 4-1 and formulation 4-2, respectively, with the result that no leak was observed from the filled substances at all.

### Experiment Example 1: Investigation into the compatibility in cast films

Screening was carried out to determine a high-molecular compound which is suited as a sealing solution (solution for preparing the band-seal) for PONDAC capsules. As a compound which may possibly be compatible with PONDAC, investigation was conducted on Plasdon S-630 which has a structure similar to constituents of PONDAC, polyvinylpyrrolidone (PVP) and polyethylene glycol (tradename of POLYOX, produced by Dow Chemical Co.), as well as hydroxypropyl methylcellulose (HPMC) and methylcellulose (MC) which are in extensive use as a water-soluble coating base for tablets.

There were prepared the solution for preparing the band-seal whose compositions are shown in below Table 2, and each of the solutions was cast onto a glass plate applied on its surface with lecithin with use of an applicator for preparation of cast films. After being dried through aeration at room temperature, the resultant cast film was visually observed for any phase-separation. The figures indicated in Table 2 are expressed on a % by weight basis.

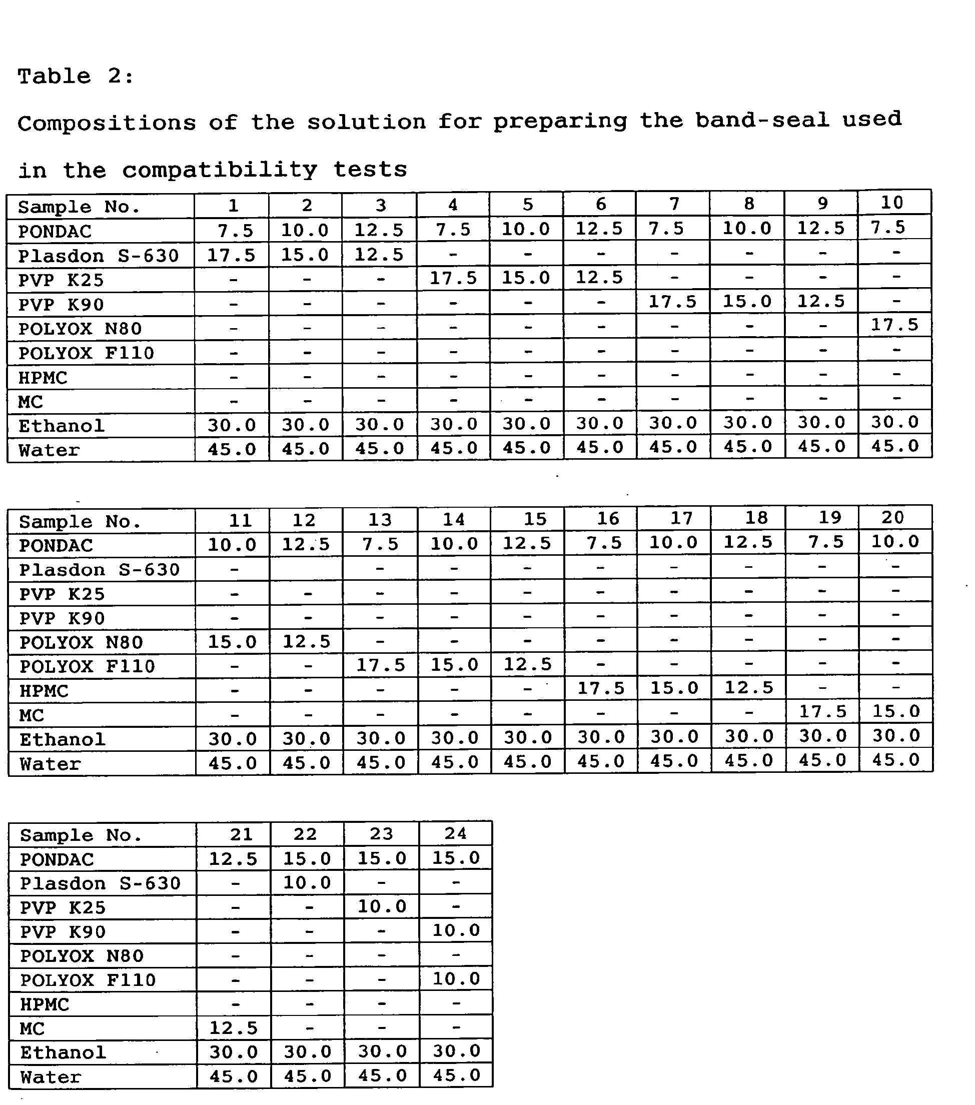

### Results:

Table 3 tabulates the results of the test on the compatibility of various high-molecular compounds with the PONDAC capsule.

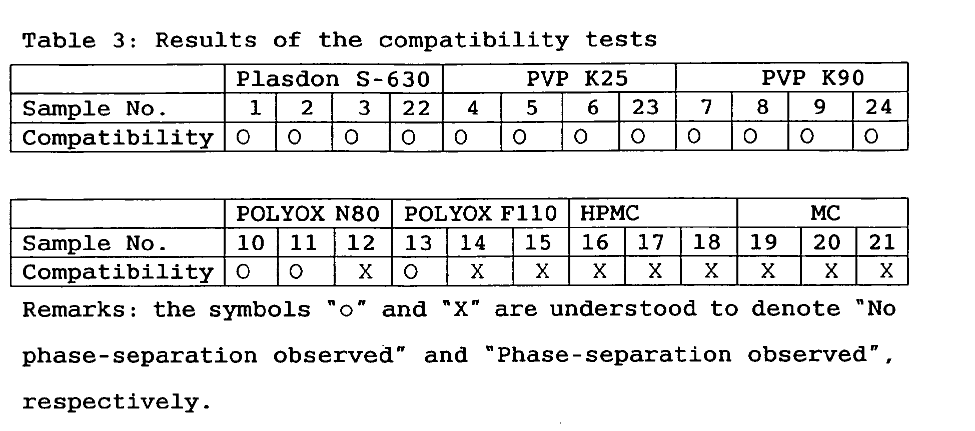

As can be seen from the above Table 3, Plasdon S-630, PVP K25 and PVP K90 were not observed to develop any phase separation with PONDAC, and were found to be suited as an ingredient for the sealing solution, whereas POLYOX N80, POLYOX F110, HPMC and MC were observed to develop phase separation, leading to the conclusion that they are not suited as an ingredient for the sealing solution.

### Experiment Example 2: Investigation into the viscosity suitability of a 25% solution for preparing the band-seal

Plasdon S-630, PVP, POLYOX and PONDAC were examined for the viscosity suitability, when converted into the sealing solutions (the solution for preparing the band-seal) whose compositions are shown in below Table 4, with the results being tabulated in Table 5, whereby the viscosity was measured as a 25% solution for preparing the band seal each of high-molecular compound(s) in the water-alcohol mixed solution with a B-type viscometer (40°C).

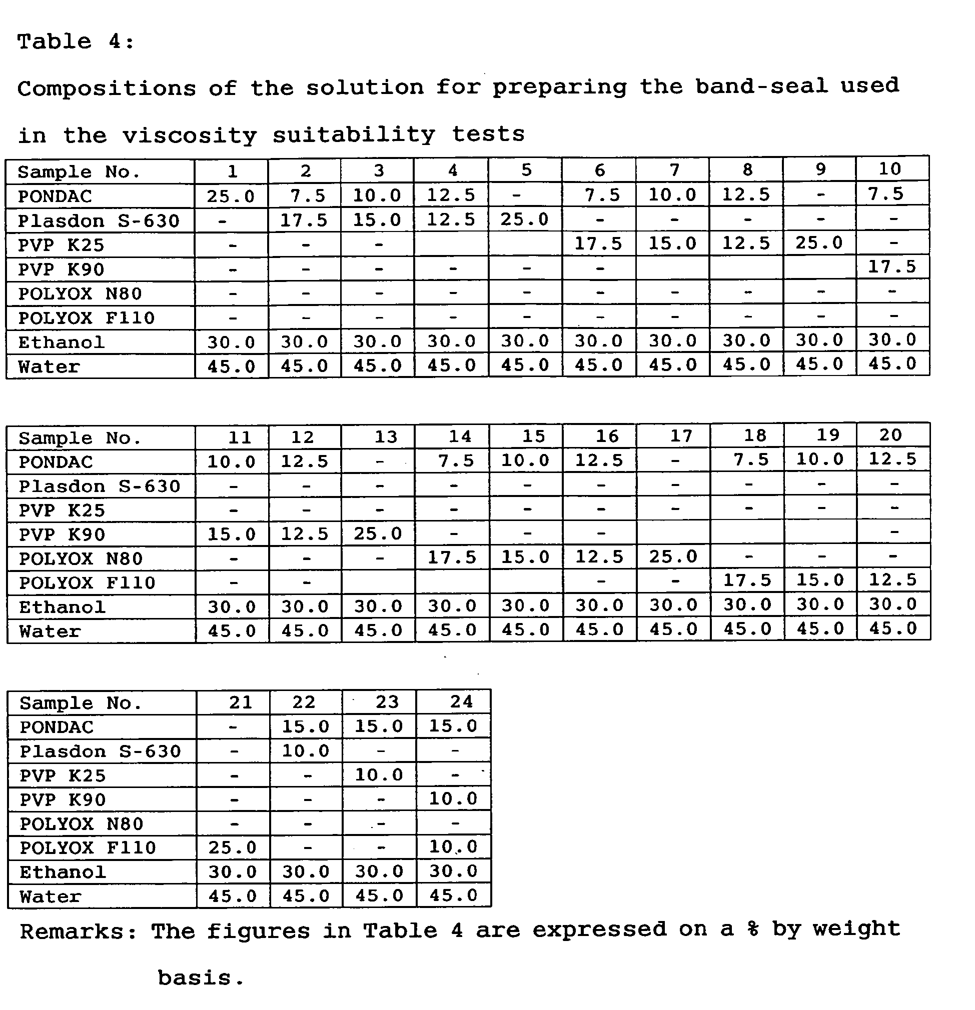

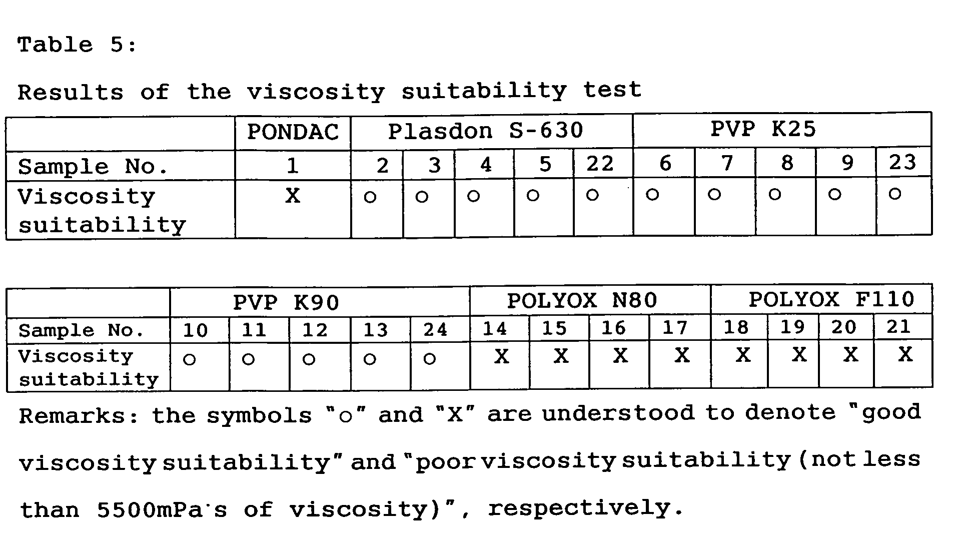

As is evident from above Tables 4 and 5, a 25% solution for preparing the band-seal each of Plasdon S-630, PVP K25 and PVP K90, showing a viscosity of less than 5500mPa·s, were judged to be suited, whereas PONDAC alone and POLYOX based high-molecular compounds provided 25% solution for preparing the band-seal with increased viscosities, all of which failed to seal capsules uniformly, and were judged not to be suited.

### Experiment Example 3

Two cast films (measuring by ca. 100µm thick x 50mm length x 10mm wide) of PONDAC each were put on the other as illustrated in Fig. 1, and the sealing solution was applied onto the portion encircled by a dotted line (adhered area: 10mm x 10mm) , followed by drying for ca. 60 min. The film was assessed and ascertained for adhesiveness by visually observing the appearance for crackings and peelings and through procedural operations, such as grabbing of both ends of the film to make slight detachment. Table 6 as presented below show the compositions of the sealing solutions used in the adhesiveness test, wherein the figures are expressed on a % by weight basis.

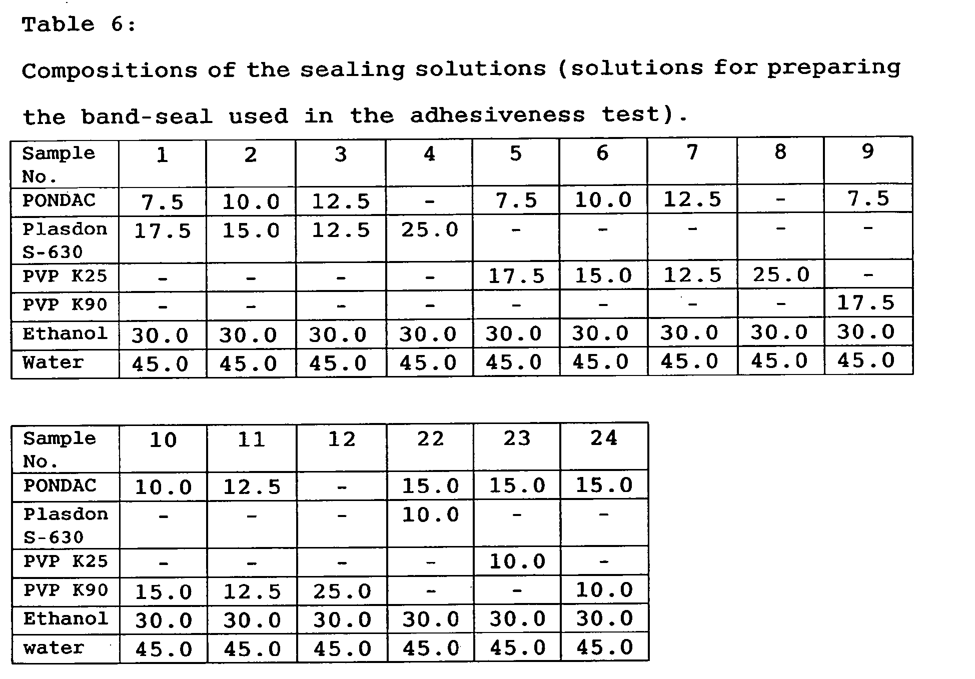

Table 7 tabulates the results of the adhesiveness test with the sealing solution (solution for preparing the band-seal) showing the compositions as tabulated in above Table 6.

**Table 7:**

| Results of the adhesiveness test | | | | | | | | | | | | | | | |
|---|---|---|---|---|---|---|---|---|---|---|---|---|---|---|---|
| | Plasdon S-630 | | | | | PVP K25 | | | | | PVP K90 | | | | |
| Sample No. | 1 | 2 | 3 | 4 | 22 | 5 | 6 | 7 | 8 | 23 | 9 | 10 | 11 | 12 | 24 |
| Adhesiveness | o | o | o | o | o | o | o | o | Δ | o | o | o | o | o | o |

| | | | | | | | | | | | | | | | |
|---|---|---|---|---|---|---|---|---|---|---|---|---|---|---|---|
| Remarks: The symbols "o" and "Δ" are understood to denote "Good adhesiveness" and "No peeling of the film found, and cracking observed on the band-sealed portion", respectively. | | | | | | | | | | | | | | | |

As is evident from above table 7, Plasdon S-630 and PVP K90 exhibited good adhesiveness, while PVP K25 provided the solutions having contents of 17.5, 15.0, 12.5 and 10 .0% by weight with improved adhesiveness, although PVP K25 when incorporated into the solution at a content of 25% by weight, yielded the band-sealed portion with crackings but freed of peeling.

### Reference Example: Production of a polyvinyl alcohol copolymer used in the band-seal

Charged into a separable flaks equipped with a cooling-refluxing condenser, dropping funnel, thermometer, nitrogen-gas introducing tube and stirrer were 175.8 g of a polyvinyl alcohol (PVA) (EG05, a degree of polymerization of 500, a degree of saponification of 88%, produced by Nippon synthetic Chemical Co. of Japan) and 582.3 g of ion-exchanged water,followed by dispersion at ambient temperature and complete dissolution at 95°C. Then, 5.4 g of acrylic acid and 37.3 g of methyl methacrylate were added, and after flushing with nitrogen gas, the temperature was raised up to 50°C. Subsequently, 8.5 g of tert-butyl hydroperoxide and 8.5 g of sodium erythorbate were added, and the reaction was continued for 4 hours to give a PVA copolymer. The resultant copolymer was dried and pulverized in accordance with the ordinarily employed procedures to give powdered PVA copolymer.

### Industrial Applicability

The solution for preparing the band-seal according to the present invention is extremely useful for leak-tight, stable sealing of hard capsules intended for use in pharmaceuticals, healthy foods, foods, etc., and the thus-produced capsules can withstand transportation and storage for a prolonged period of time.

## Claims

1. A band-seal intended for use in the hard capsule comprising a polyvinyl alcohol copolymer or pullulan as a base,
**characterized in that** the band-seal is incorporated with at least one or not less than two of the below-described (a) to (c):
(a) A polyvinylpyrrolidone with a molecular weight of 100, 000 to 4,000,000;
(b) A polyvinylpyrrolidone with a molecular weight of 10,000 to 80,000 in the proportion of not more than 90% by weight against the total weight of the band-seal; and
(c) A copolymer from 1-vinyl-2-pyrrolidone and vinyl acetate.

2. The band-seal according to Claim 1, wherein the polyvinyl alcohol copolymer is contained in the band seal at a proportion of not more than 60% by weight against the total weight of the band seal.

3. A solution for preparing a band-seal intended for use in a hard capsule comprising a polyvinyl alcohol copolymer or pullulan as a base, wherein the solution for preparing a band-seal comprises at least one or not less than two of the below-described (a) to (c):
(a) A polyvinylpyrrolidone with a molecular weight of 100,000 to 4,000,000;
(b) A polyvinylpyrrolidone with a molecular weight of 10,000 to 80,000; and
(c) A copolymer from 1-vinyl-2-pyrrolidone and vinyl acetate, and (a) and (c) are contained in the proportion of 3 to 35% by weight against the total weight of the solution for preparing the band-seal and (b) is contained in the proportion of 8 to 40% by weight against the total weight of the solution for preparing the band-seal.

4. The solution for preparing the band-seal according to Claim 3, wherein the polyvinyl alcohol copolymer is contained in the proportion of not more than 15% by weight against the total weight of the solution for preparing the band-seal.

5. The solution for preparing the band-seal according to Claim 3 or 4, wherein the said solution for preparing the band-seal shows a viscosity of 100 to 5,500 mPa·s.

6. The band-seal or the solution for preparing the band-seal according to any one of Claims 1 to 3, **characterized in that** the copolymer from 1-vinyl-2-pyrrolidone and vinyl acetate is composedof 1-vinyl-2-pyrrolidone and vinyl acetate at the weight ratio of 4:1 to 1:4.

7. The band-seal or the solution for preparing the band-seal according to Claim 2 or 4, **characterized in that** the polyvinyl alcohol copolymer is produced by copolymerizing a partially saponified polyvinyl alcohol with a mean degree of polymerization of 300 to 500 and a polymerizable vinyl monomer at the weight ratio of 6:4 to 9:1, with the polymerizable vinyl monomer being composed of acrylic acid and methyl methacrylate at the weight ratio of 3:7 to 0.5:9.5.

8. The band-seal or the solution for preparing the band-seal according to Claim 2 or 4, **characterized in that** the polyvinyl alcohol copolymer is obtained by copolymerizing a partially saponified polyvinyl alcohol with a mean degree of polymerization of 300 to 500 with methyl methacrylate and acrylic acid at the weight ratio of 60 to 90:7 to 38:0.5 to 12.

9. A hard capsule which is sealed with the band-seal according to any one of Claims 1, 2, and 6 to 8.

10. A method for sealing a hard capsule, **characterized in that** said method comprises sealing a hard capsule with the band-seal according to any one of Claims 1, 2, and 6 to 8.

11. A hard capsule which has a liquid-formed or solid-formed substance filled therein, followed by sealing with the band-seal according to any one of Claims 1, 2, and 6 to 8.

12. A hard capsule which has a liquid-formed or solid-formed drug substance filled therein, followed by sealing with the band-seal according to any one of Claims 1, 2, and 6 to 8.

13. A hard capsule, **characterized in that** said hard capsule is produced by filling a liquid-formed or solid-formed substance therein and sealing the hard capsule with the solution for preparing the band-seal according to any one of Claims 3 to 8, followed by drying.

14. A process for producing a hard capsule preparation, **characterized in that** said process comprises filling a liquid-formed or solid-formed substance in a hard capsule, and sealing the hard capsule with the solution for preparing the band-seal according to any one of Claims 3 to 8, followed by drying.
